# EUROPEAN PATENT APPLICATION

(11) **EP 1 068 838 A2**
(43) Date of publication of application: **17.01.2001**
(21) Application number: 00115280.0
(22) Date of filing: 14.07.2000
(51) Int. Cl.: A61B 19/00

(54) **Reclining chair for medical use**

(30) Priority: 16.07.1999 IT BO990403
(71) Applicant: Lenzi, Gino, 40033 Casalecchio Di Reno (Bologna) (IT)
(72) Inventor: Lenzi, Gino, 40033 Casalecchio Di Reno (Bologna) (IT)
(74) Representative: Negrini, Elena

(57) **Abstract**

A reclining chair for medical use, having a support base (2) for supporting a post (3), to which is connected a seat (4) and a backrest (5), includes:
- support means (6), located between the post (3) and the seat (4) and having one end connected to said post (3) and the remaining end is connected to the front end of sitting members (7) of the seat (4);
- bracket means (8) having one end connected near the rear end of the sitting member (7) and supporting the backrest (5) in correspondence of the remaining end;
- first actuator means (9), located between the support means (6) and the sitting members (7), allowing to vary the angle between the support means (6) and the sitting members (7) from a lowered condition (A) to a raised condition (B) in which the seat (4) is respectively reclined back or forth in such a way that the front end keeps almost its same height.

## Description

The present invention relates to seating devices, in particular refers to a reclining chair for medical-sanitary use which is preferably used by the dentists.

The known dentist chairs have essentially a base with small wheels for running on the floor, a telescopic supporting post for regulating the height of the seat pivoted horizontally since it is almost centrally hinged to the post, and a backrest sometimes equipped with a vertical adjustment.

So the dentist, who uses one of the known chairs, can adjust the seat height, either the back and/or forward seat inclination, or sometimes the backrest height, or also the backrest inclination when hinged to the seat.

The main drawback of the known chairs is that the user, in particular the dentist, although can adjust with precision the seat and also the chair's backrest, every time in particular when he changes the sitting inclination, unavoidably changes also the height and the inclination of his knees with consequence of uncomfortable and incorrect leg postures of the dentist. In other words, the user of a known chair, due to professional reasons, is obliged to adjust several times per day the seat height or inclination, so that he must always operate the two adjustments contemporarily in order to get the correct position with respect to the patient. These continuous adjustments depend mainly on the fact that the dentist knees changes their height in consequence of the chair movement and therefore the body position of the dentist changes also.

The object of the present invention is to propose a reclining chair, preferably for dentists, which is tilting without moving in any way the knees height of the user sitting thereon, also keeping the seat height, as well as the backrest height and inclination.

Further object is to propose a chair allowing the backrest and seat movement by means of only a control lever.

Further object of the present invention is to propose a chair of simple production, of lower cost and of high reliability.

The above mentioned objects are achieved according to the content of the claims.

The features of the invention are shown as following with reference to the enclosed drawings, in which:
- figure 1 shows a side view of the chair, object of the invention, in which some parts are removed away for better underlining others;
- figure 2 shows a front view of the chair in figure 1;
- figure 3 shows a top view of the chair in figure 1;
- figures 4 and 5 show respectively extreme conditions in which backrest and chair in figure 1 are reclined back and forth, and the seat is respectively at the lowest and highest position;
- figure 6 shows a schematic enlarged view of the chair in figure 1 in its reclined back and forth condition, the latter is shown with dash-line.

With reference to figures from 1 to 5, numeral 1 refers to a chair essentially comprising a support base 2, a seat 4, a backrest 5, support means 6, sitting members 7, bracket means 8, actuator means, first 9, second 10 and third 18, elastic means 11 and adjustment means 12.

The support base 2 comprises a circular ring 2a connecting a plurality of wheels 17, the quantity is five according to the preferred embodiment, fit for allowing the whole chair to run on the floor. Each wheel is rotationally blocked to a radial support fixed at the base of a post 3 of the support base 2.

The post 3 is essentially constituted by a base 3a, housing the third actuator means 18 comprising a cylinder whose stem 3b slides into the base in which the post is housed partially.

The support means 6 is located between the stem 3b and the seat 4 and has one end connected to the free end of the stem 3b, while the remaining end of the support means 6 is hinged to the front end of the sitting members 7 of the seat 4.

Said sitting members 7 are constituted by a shaped plate and comprise three elements interconnected in such a way to constitute an upwards overturned concavity housing and fit to constitute the support base of the seat.

In the preferred embodiment, the seat 4 comprises a padding having a horse saddle shape.

The bracket means 8 comprises essentially a bracket 8a, "L" shaped, one end of which is hinged to the lower wall of the sitting members 7 in correspondence of the interconnection between the central element and rear element of the shaped plate, while the remaining end supports the backrest 5.

The adjustment means 12 is located between the bracket 8a and the backrest 5 and comprises a plate 13 fixed to the free end of the bracket 8a and supporting slidingly a carriage 14 to which is connected a support plate 15 of the backrest 5.

In the preferred embodiment of the invention, the plate 15 of the backrest 5 essentially comprises a pair of side plates, 15a and 15b, connected to the carriage 14 by means of bolts 19 with spring therein.

The plates 15a and 15b are coated with related lobed paddings, respectively 5a and 5b, while the bracket means 8 is covered by a covering 20.

The first actuator means 9 is positioned between the support means 6 and the sitting members 7 and comprises essentially a pneumatic cylinder whose one end is hinged in proximity of the front end of the support means 6, while the piston rod is hinged to the middle element of the sitting members 7.

The second actuator means 10 is positioned between the sitting members 7 and the bracket means 8 and comprises essentially a pneumatic piston whose one end is hinged in correspondence of the bracket 8a vertex, while the piston rod is hinged in correspondence of the interconnection between the middle and the rear elements of the sitting members 7.

The third actuator means 18 is located inside the post 3 and essentially consists of a pneumatic cylinder.

The lever means 16 is hinged to the sitting members 7 and has an end connected to the respective drive levers of the actuator means, first 9, second 10 and third 18.

In the preferred embodiment, the elastic means 11, positioned between the sitting members 7 and the support means 6, is constituted by a spring 11a, one of its ends is fixed to the rear element of the sitting members 7 and the remaining end is connected to the extremity of screw members 11b connected close to the front end of the support means 6 and having a knob 11c in correspondence of the free end.

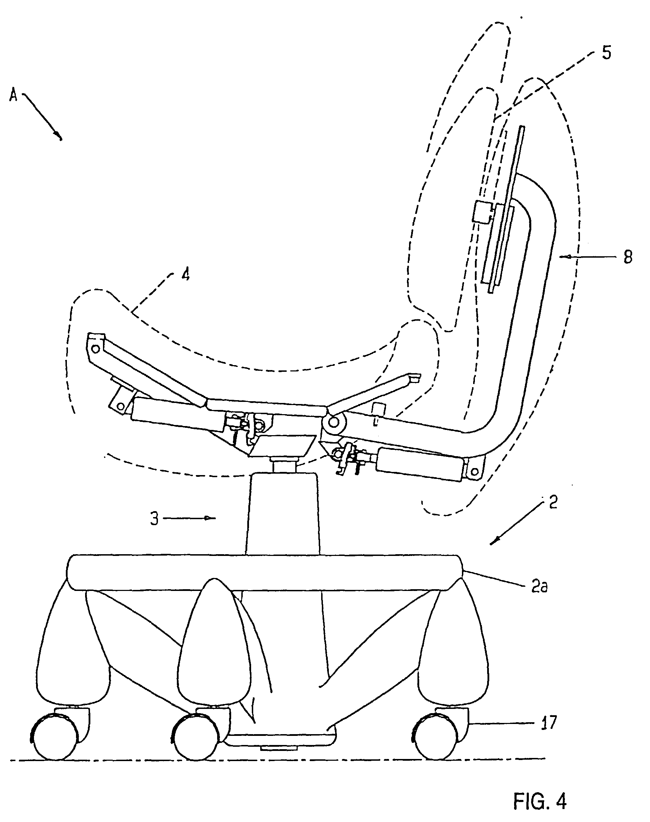

With particular reference to figures 4-6, the usage of said chair 1 is very simple and the user, in particular a dentist, can regulate the chair height from the ground, the inclination of the seat 4 as well as the height and the inclination of the backrest 5.

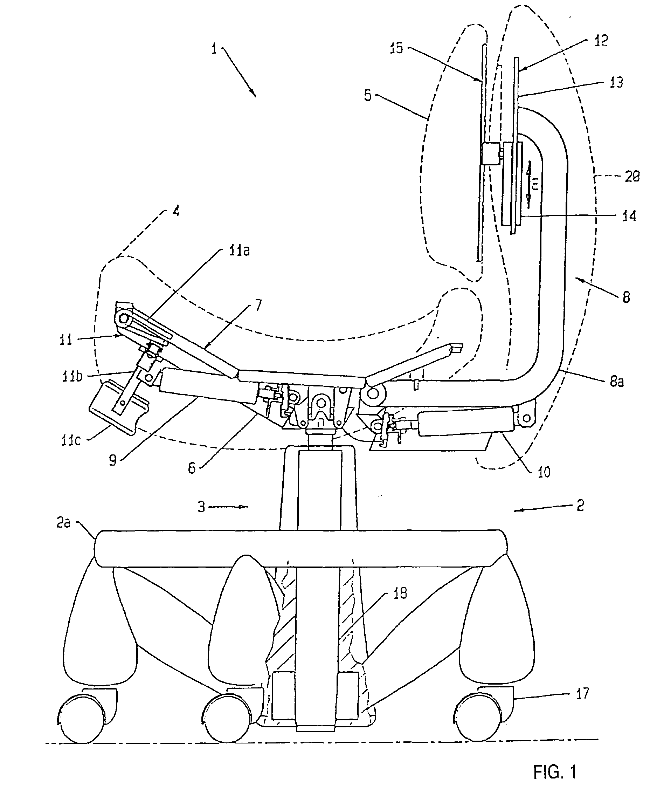

Once the user sits down on the chair 1, firstly regulates the height of the backrest 5 by stepping moving the carriage 14 according to the double arrow in figure 1 that, by virtue of the friction between the carriage 14 and the plate 13, allowing this latter to keep the pre-selected position without blocking the carriage 14 to the plate 13. So the user can operate either the actuators, first 9 and second 10, respectively by rotating backwards and forwards the free end of the lever 16, or can operate the third actuator means 18 by moving vertically the same lever.

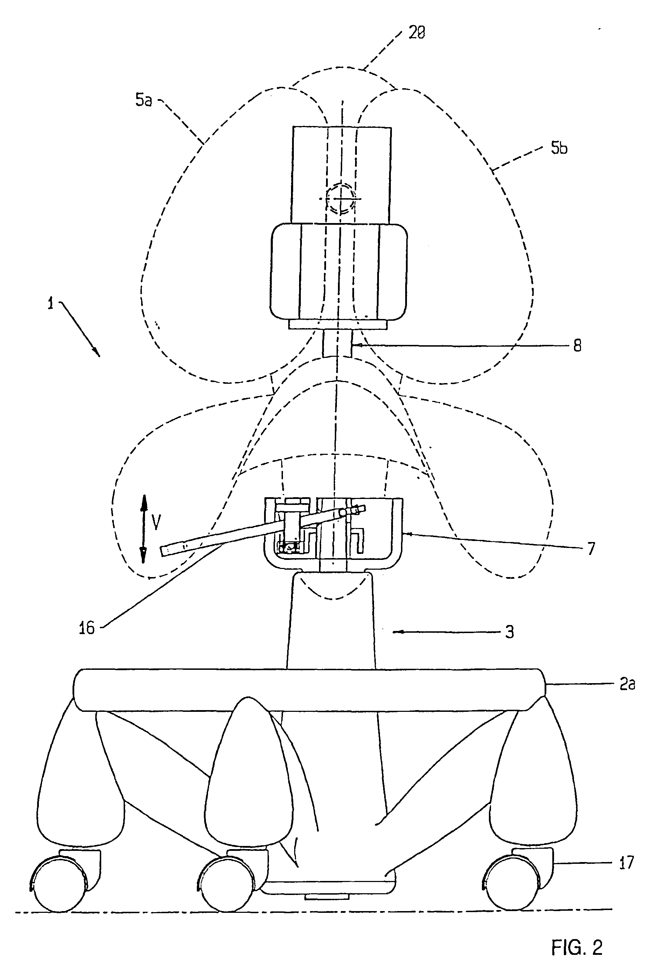

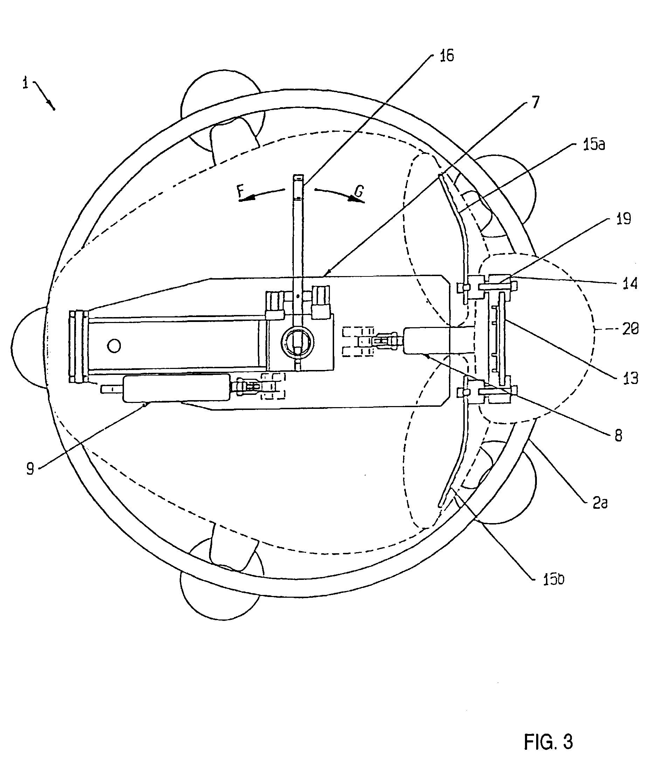

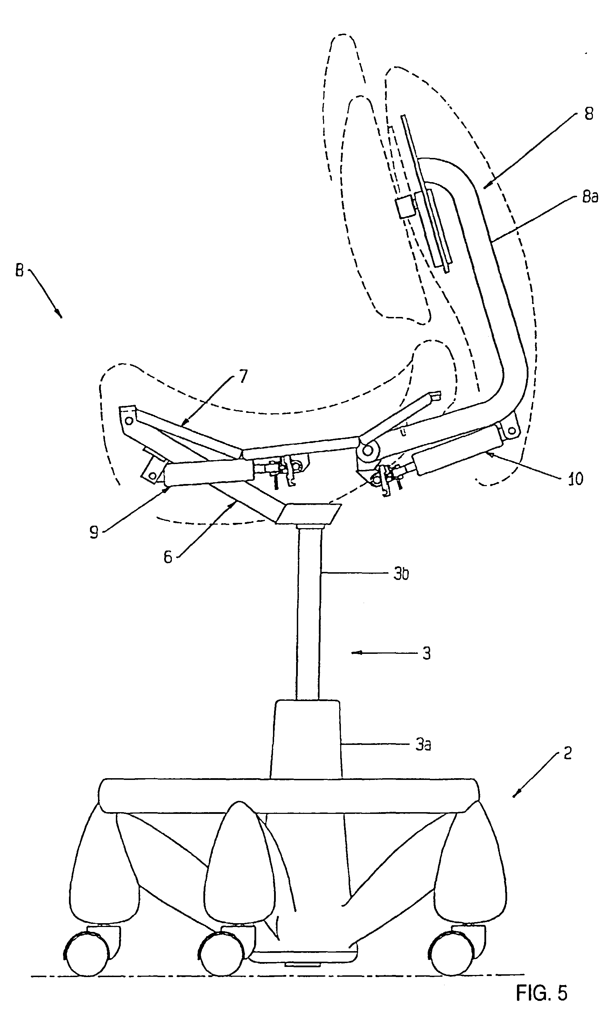

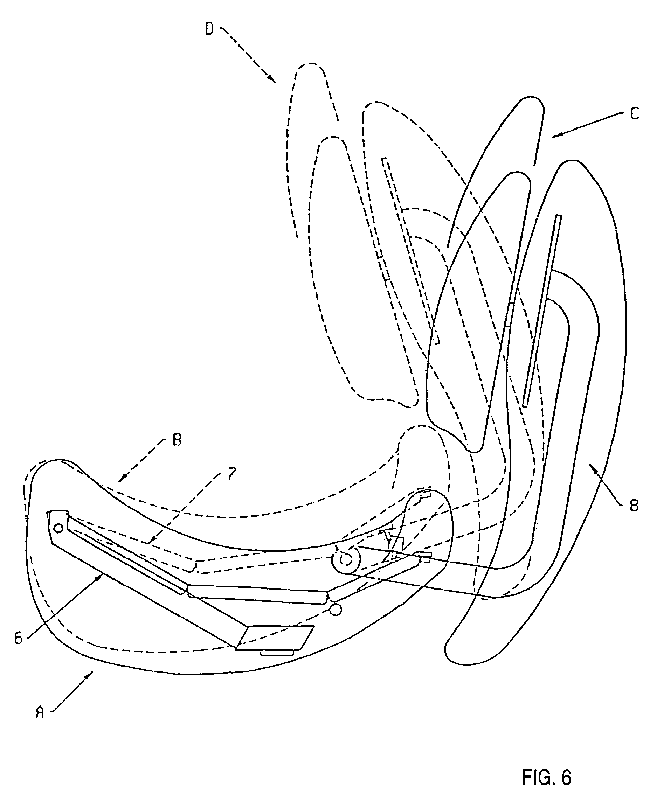

In particular, the vertical movement of the lever 16, according to the double arrow V in figure 2, allows to lower the seat 4 completely, as shown in figure 4, or to lift it completely, as shown in figure 5, while the forth rotation, according to the arrow F in figure 3, of the free end of the lever 16 allows the variation of the angular disposition between the support means 6 and the sitting members 7, between a lowest position A or an highest position B, respectively shown in figures 4 and 5, in which the seat 4 is respectively reclined back or forth in such a way that the front end remains almost to its same height, as shown clearly in figure 6.

Always acting on the free end of the lever 16, the backward rotation of this latter, according to the arrow G in figure 3, allows to vary the angle between the sitting members 7 and the bracket means 8 from a reclined condition C to a forward condition D, shown in figure 6, in which the backrest 5 has respectively back and forth reclined end positions.

The user can furthermore make one more regulation acting on the elastic means 11 that, acting on the spring 11a, proportionally contrasts the different user's weight acting on the actuator means, first 9, second 10 and third 18.

It can advantageously be noted that, once the dentist has made all the wished adjustments, he can change the chair height, the backrest height and inclination, the seat and back height and inclination simply acting only on a lever 16. Besides, the movement of the reclining seat 4, from the lowest position A to the highest position B and vice versa, occurs without variation of the user's knees height.

The main advantage of the present invention is to provide reclining chair fits to recline the seat without moving the knees height of the user sitting on the chair, also keeping the adjusted height of the seat and the backrest, as well as the inclination of this latter.

A further advantage is to provide a chair allowing the movement of the backrest and the seat by means of only an actuator lever.

Further advantage of the present invention is to provide a chair of simple production, of lower cost and of high reliability.

## Claims

1. Reclining chair for medical use having a support base (2) for supporting a post (3), to which is connected a seat (4) and a backrest (5), said chair (1) characterized in that includes:
- support means (6) located between said post (3) and said seat (4) and having one end connected to said post (3) and the remaining end connected to the front end of sitting members (7) of said seat (4);
- bracket means (8) having one end connected near the rear end of said sitting member (7) and supporting said backrest (5) in correspondence of the remaining end;
- first actuator means (9), located between said support means (6) and said sitting members (7), allowing to vary the angle between said support means (6) and said sitting members (7) from a low condition (A) to a high condition (B) in which said seat (4) is respectively reclined back or forth in such a way that the front end remains almost at its same height.

2. Chair according to claim 1 characterized in that furthermore comprises second actuator means (10), positioned between said sitting members (7) and said bracket means (8), allowing the angular variation between said sitting members (7) and said bracket means (8) from a back reclined condition (C) to a forward reclined condition (D) in which said backrest (5) has respectively back and forth reclined positions.

3. Chair according to claim 1 characterized in that further comprises elastic means (11), positioned between said sitting members (7) and said support means (6), fit to contrast proportionally the different user weight acting onto said actuator means, first (9), second (10) and third (18).

4. Chair according to claim 3 characterized in that said elastic means (11) consists of a spring (11a) whose one first end is fixed to said sitting members (7) and the remaining end is connected to said support means (6) with interposition of adjustable screw members (11b).

5. Chair according to claim 1 characterized in that further includes adjustment means (12) interposed between said bracket means (8) and said backrest (5) comprising a plate (13) fixed to the free end of said bracket means (8) and slidingly supporting a carriage (14) to which is connected at least one plate (15) for supporting said backrest (5), said adjustment means (12) fit for allowing the distance adjustment between said backrest (5) and said seat (4).

6. Chair according to claim 1 characterized in that said backrest (5) is essentially constituted by a pair of plates (15a, 15b) connected to said bracket means (8), said plates (15a, 15b) being covered by related padding (5a, 5b).

7. Chair according to claim 1 characterized in that said post (3) is telescopic and comprises third actuator means (18).

8. Chair according to claims 2 and 7 characterized in that further includes lever means (16) hinged to said sitting members (7) and having one end connected to said actuator means, first (9), second (10) and third (18), said lever means (16) designed to activate said actuator means, first (9) and second (10), respectively by rotating back and forth the free end of said lever means (16), and said third actuator means (18) by moving vertically the free end of said lever (16).

9. Chair according to claims 2 and 7 characterized in that said actuator means, first (9), second (10) and third (18), are pneumatic cylinders.

10. Chair according to claim 2 characterized in that further includes lever means (16) hinged to said sitting members (7) and having one end connected to said actuator means, first (9) and second (10), said lever means (16) fit for operating said actuator means, first (9) and second (10), respectively by rotating back and forth the free end of said lever means (16).

11. Chair according to claim 1 characterized in that said support base (2) is provided with a plurality of wheels (17).

12. Chair according to claim 1 characterized in that said seat (4) comprises a saddle shaped padding and said backrest (5) comprises a padding having two lobes, respectively (5a), (5b).
